# EUROPÄISCHE PATENTANMELDUNG

(11) **EP 1 541 568 A1**
(43) Veröffentlichungstag der Anmeldung: **15.06.2005**
(21) Anmeldenummer: 03028224.8
(22) Anmeldetag: 09.12.2003
(51) Int. Cl.: C07D 317/36

(54) **Reaktive cyclische Carbonate und Harnstoffe zur Modifizierung von Biomolekülen, Polymeren und Oberflächen**

(71) Anmelder: Deutsches Wollforschungsinstitut an der Rheinisch-Westfälischen Technischen Hochschule Aachen e.V., 52062 Aachen (DE)
(72) Erfinder: Keul, Helmut, 52074 Aachen (DE); Möller, Martin, 52070 Aachen (DE); Pasquier, Nicolas, 1630 Bulle (CH); Ubaghs, Luc, 6224 JP Maastricht (NL)
(74) Vertreter: Reitstötter - Kinzebach & Partner (GbR)

(57) **Zusammenfassung**

Beschrieben werden reaktive cyclische Carbonate und Harnstoffe der Formel I oder II 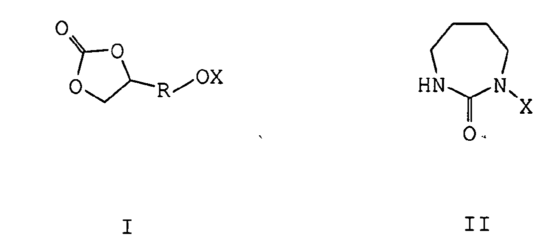 worin R und X die in der Beschreibung angegebene Bedeutung haben. Sie gestatten die gezielte Einführung funktionaler Gruppierungen in Biomoleküle, Polymere und Oberflächen unter milden Bedingungen.

## Beschreibung

Die vorliegende Erfindung betrifft reaktive cyclische Carbonate und Harnstoffe zur Modifizierung von Biomolekülen, Polymeren und Oberflächen, Umsetzungsprodukte der cyclischen Carbonate und Harnstoffe mit bestimmten Polymeren und ein Verfahren zur Modifizierung von Biomolekülen, Polymeren und Oberflächen.

Die dauerhafte Funktionalisierung von Materialoberflächen ist für eine Vielzahl von Materialien und Anwendungen von Bedeutung. Beispielsweise können Materialoberflächen durch Beschichtung mit Heparin biokompatibel gemacht werden. Andere Anwendungen sind schmutzabweisende und bakteriostatische Ausrüstungen und die Verbesserung der Haftung von Klebstoffen und Lacken.

Die Modifikation einer Oberfläche kann entweder reaktiv durch Ausbildung kovalenter Bindungen oder durch Chemisorption bzw. Adsorbtion erfolgen. Beispiele für eine reaktive Modifizierung sind solche mit Isocyanaten, mit Silanen und durch radikalische Pfropfreaktionen. Ein häufig angewandtes Verfahren zum Einführen funktionaler Gruppen an Oberflächen bedient sich der Beschichtung mit funktionalen Silanen, insbesondere Aminosilanen. Die Behandlung mit Aminosilanen ist technisch aufwändig und kann nicht aus wässriger Lösung erfolgen.

Die Chemisorption beruht auf ionischen Wechselwirkungen zwischen negativen Oberflächenladungen und kationischen Verbindungen, z. B. Ammoniumverbindungen. An der Oberfläche fast aller Materialien findet man negativ geladene Gruppen. Bei hydrophoben Polymeren, wie den Polyolefinen, bilden sich negative Oberflächenladungen durch Oxidation an Luft; diese kann künstlich durch eine Plasmabehandlung oder UV-Oxidation verstärkt werden. Polykationische Polymere, wie Polyammoniumverbindungen, adsorbieren auf solchen Oberflächen durch Polyelektrolytkomplexbildung. Durch die Kooperativität der vielfachen Ionenpaarbildung entsteht eine sehr starke Bindung, die selbst bei hohen Ionenstärken und extremen pH-Werten stabil ist.

Auch die gezielte Modifizierung von Biomolekülen ist in vielen Fällen wünschenswert. So kann die biologische Halbwertszeit verschiedener Wirkstoffe beispielsweise durch Anfügen von Polyoxyalkylenresten verbessert werden.

Es besteht ein Bedarf an Reagenzien und Verfahren, die es gestatten, gezielt bestimmte chemisch funktionale Gruppierungen in Biomoleküle und Oberflächen einzuführen. Die beteiligten Umsetzungen sollten unter milden Bedingungen und, soweit Biomoleküle beteiligt sind, ohne deren Denaturierung ablaufen.

Die US-A 5,650,234 beschreibt gemischte Polyethylenglykolcarbonate, die mit Aminogruppen in Aminoglykanen oder Proteinen bzw. Aminogruppen-haltigen Oberflächen glatt reagieren. Die Polyethylenglykolcarbonate gestatten die kovalente Bindung von Biomolekülen an Oberflächen.

Weiterhin besteht ein Bedarf nach leicht zugänglichen polykationischen Verbindungen zur Oberflächenbehandlung, die aus wässriger Lösung aufgebracht werden können und bestimmte funktionale Gruppen enthalten bzw. eine anschließende weitere Modifikation erlauben.

Die Erfindung betrifft in einem ersten Aspekt Verbindungen der allgemeinen Formel I oder II 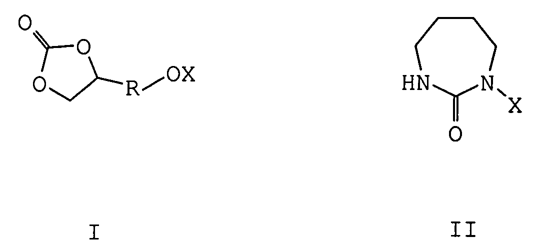 worin
R für C₁-C₁₂-Alkylen, vorzugsweise C₁-C₄-Alkylen, insbesondere Methylen, steht;
X für CO-NH-R¹ steht; und
R¹ für C₁-C₃₀-Alkyl, C₁-C₃₀-Halogenalkyl, C₁-C₃₀-Hydroxyalkyl, C₁-C₆-Alkyloxy-C₁-C₃₀-alkyl, C₁-C₆-Alkylcarbonyloxy-C₁-C₃₀-alkyl, Amino-C₁-C₃₀-alkyl, Mono- oder Di(C₁-C₆-alkyl)amino-C₁-C₃₀-alkyl, Ammonio-C₁-C₃₀-alkyl, Polyoxyalkylen-C₁-C₃₀-alkyl, Polysiloxanyl-C₁-C₃₀-alkyl, (Meth)acryloyloxy-C₁-C₃₀-alkyl, Sulfono-C₁-C₃₀-alkyl, Phosphono-C₁-C₃₀-alkyl, Di(C₁-C₆-alkyl)phosphono-C₁-C₃₀-alkyl, Phosphonato-C₁-C₃₀-alkyl, Di(C₁-C₆-alkyl)phosphonato-C₁-C₃₀-alkyl oder einen Saccharidrest steht.

Der Begriff "Alkyl" soll geradkettige, verzweigte und cyclische Alkylgruppen mit 1 bis 30, vorzugsweise 1 bis 18, insbesondere 1 bis 12 Kohlenstoffatomen umfassen.

Der Begriff "Halogenalkyl" bedeutet eine Alkylgruppe, in der ein oder mehrere oder alle Wasserstoffatome durch Halogen, insbesondere Fluor, ersetzt sind. Der Begriff "Hydroxyalkyl" bedeutet eine Alkylgruppe, in der ein oder mehrere Wasserstoffatome durch Hydroxygruppen ersetzt sind. In gleicher Weise bedeuten die Begriffe "Alkyloxyalkyl", "Alkylcarbonyloxyalkyl", "Aminoalkyl", "Ammonioalkyl", "Polyoxyalkylenalkyl", "Polysiloxanylalkyl", "(Meth)acryloyloxyalkyl", "Sulfonoalkyl", "Phosphonoalkyl" und "Phosphonatoalkyl" einen Alkylrest, in dem ein oder mehrere Wasserstoffatome durch eine Alkyloxygruppe, eine Alkylcarbonyloxygruppe, eine Aminogruppe, eine Ammoniogruppe (NR³₃⁺; worin jedes R³ unabhängig z. B. für C₁-C₁₈-Alkyl oder Benzyl steht), einen Polyoxyalkenylrest, einen Polysiloxanylrest, eine (Meth)acryloyloxygruppe, eine Sulfonsäuregruppe (SO₃H), eine Phosphonsäuregruppe (PO₃H₂) oder Phosphorsäureestergruppe (OPO₃H₂) ersetzt sind. Wenn R¹ für Ammonioalkyl steht, ist die Verbindung von einem Äquivalent eines Anions, vorzugsweise eines physiologisch verträglichen Anions, wie eines Halogenids, z. B. Chlorid oder Bromid, Sulfat, Hydrogensulfat, Methosulfat, Nitrat oder dergleichen begleitet.

Der Polyoxyalkylenrest leitet sich vorzugsweise von Etylenoxid und/oder Propylenoxid ab, insbesondere von Ethylenoxid. Er kann am distalen Ende beispielsweise durch eine Hydroxy-, Alkyloxy- oder Alkaryloxygruppe terminiert sein. Der Polysiloxanylrest leitet sich vorzugsweise von Polydimethylsiloxanen ab.

Bei dem Saccharidrest handelt es sich z. B. um einen Glucosylrest.

Der Begriff "Biomolekül" umfasst alle Moleküle, die aus biologischen Systemen isoliert sind und/oder mit biologischen Systemen oder Teilen davon in Wechselwirkung treten können. Hierzu zählen vor allem Peptide, Proteine, Proteoglykane, Enzyme, Markierungsstoffe, Antikörper, Rezeptormoleküle, Antigene, Wirkstoffe. Spezielle Beispiele sind Heparin, Gewebeplasminogenaktivator, Streptokinase, Prostaglandine und dergleichen.

In bevorzugten Ausführungsformen steht R¹ für
-(CH₂)ₙ-CH₃,
-(CH₂)ₙ-(CF₂)ₘ-CF₃,
-(CH₂)ₙ-[Si(CH₃)₂-O]ₚ-H,
-(CH₂)ₙ-(O-CH₂-CH₂)ₚ-O-(CH₂)ₘ-H,
-R²-OH,
-R²-NH₂,
-R²-NR³₃⁺ Y⁻,
-R²-OCO-C(R⁴)=CH₂,
-R²-SO₃H,
-R²-PO₃H₂,
-R²-OPO₃H₂
oder einen Saccharidrest,
wobei R² für C₁-C₁₈-Alkylen, R³ für C₁-C₁₈-Alkyl oder Benzyl und R⁴ für Wasserstoff oder Methyl steht,
Y für ein Äquivalent eines Anions, wie den oben genannten, steht,
n und m unabhängig voneinander für eine ganze Zahl von 0 bis 12 stehen; und
p für eine ganze Zahl von 1 bis 100, vorzugsweise 2 bis 50, steht.

Die Verbindungen können auf verschiedene Weise unter Anwendung von Standardverfahren der organischen Synthese hergestellt werden. Bevorzugte Herstellungsarten sind in den Beispielen veranschaulicht.

Die obigen Verbindungen reagieren unter milden Bedingungen mit Nukleophilen, z. B. Hydroxy- oder Aminogruppen von Biomolekülen oder Polymeren oder an Substratoberflächen, unter Ringöffnung und Ausbildung einer kovalenten Bindung, wie im nachstehenden Schema veranschaulicht ist (worin D für das Nukleophil steht und R' für den Rest des Biomoleküls, Polymers bzw. der Substratoberfläche steht; verschiedene Nukleophile D sind in der Tabelle 2 der nachstehenden Beispiele veranschaulicht):

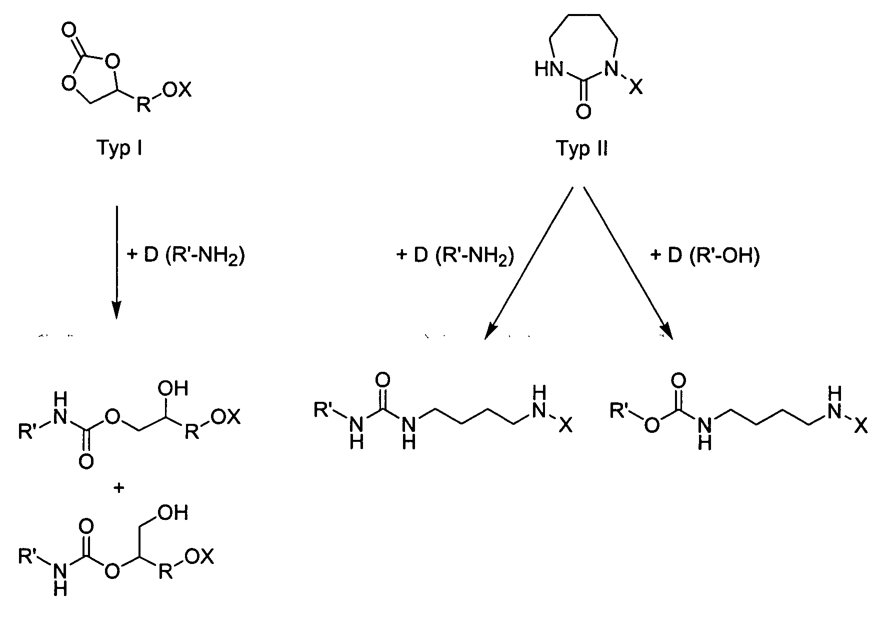

Verbindungen der Formel I reagieren dabei bevorzugt mit primären oder sekundären Aminogruppen; Verbindungen der Formel II bevorzugt mit primären oder sekundären Aminogruppen und Hydroxygruppen.

Auf diese Weise gestatten die erfindungsgemäßen Verbindungen, eine große Bandbreite verschiedener Reste X in Biomoleküle oder Polymere einzuführen bzw. an Substratoberflächen zu binden.

Die Erfindung betrifft daher außerdem ein Verfahren zur Modifizierung von Biomolekülen, Polymeren oder Oberflächen, die über funktionelle Gruppen verfügen, welche unter Hydroxygruppen, primären und sekundären Aminogruppen ausgewählt sind, bei dem man das Biomolekül, Polymer oder die Oberfläche unter Bedingungen mit einer erfindungsgemäßen Verbindung in Kontakt bringt, dass die funktionellen Gruppen unter Ausbildung einer kovalenten Bindung mit der Verbindung der Formel I oder II reagieren.

Oberflächen, die nach diesem Verfahren behandelt werden können, sind z. B. die Oberflächen von Materialien, die intrinsische Amino- und/oder Hydroxygruppen aufweisen, oder Oberflächen, die nach an sich bekannten Verfahren mit Aminosilanen behandelt wurden.

Ein weiterer Gegenstand der Erfindung sind die Umsetzungsprodukte der erfindungsgemäßen Verbindungen mit einem Polymer, das über funktionelle Gruppen verfügt, die unter Hydroxygruppen, primären und sekundären Aminogruppen ausgewählt sind. Die Umsetzung der erfindungsgemäßen Verbindungen mit einem Polyamin oder einem Polyol erlaubt die Einführung der funktionalen Gruppe X in die Polymerstruktur. Beispiele für Polyamine sind Polyvinylamin, Polyallylamin, Polyethylenimin, Chitosan, Polyamid-Epichlorhydrin-Harze, wie sie unter der Bezeichnung Hercosett® vertrieben werden, Polyaminostyrol, Peptide oder Proteine, wie Gelatine. Bevorzugte Proteine sind modifizierte Keratinpolypeptide, die durch reverse Proteolyse (Plastein-Reaktion) mit Lysin (2,6-Diaminohexansäure) angereichert sind.

Bei der Umsetzung des Polymers mit den erfindungsgemäßen Verbindungen handelt es sich um eine polymeranaloge Reaktion. Die Konzentration der funktionellen Gruppen X im Umsetzungsprodukt lässt sich durch Wahl des stöchiometrischen Verhältnisses der Amino- oder Hydroxygruppen im Polymer zum Reagenz I oder II einstellen.

In bevorzugten Umsetzungsprodukten steht zumindest ein Teil der Reste R¹ für Ammonioalkyl, d. h. es handelt sich bei den Umsetzungsprodukten um kationische Polylelektrolyte. Die erhaltenen kationischen Polylelektrolyte können auf einer anionischen Materialoberfläche durch Polyelektrolytwechselwirkung dauerhaft haften.

Bevorzugt wird die Umsetzung so ausgeführt, dass bei der Umsetzung mit den erfindungsgemäßen Verbindungen nicht alle Hydroxy- und/oder Aminogruppen des Polymers teilnehmen, so dass die Umsetzungsprodukte noch reaktive Hydroxy- und/oder primäre oder sekundäre Aminfunktionen enthalten. Auf diese Weise lassen sich reaktive NH₂-, NH- bzw. OH-Gruppen in die Oberflächen einführen, die eine schnelle und effektive Anbindung unterschiedlicher Beschichtungen erlauben. Beispiele sind Epoxide, Isocyanate, Anhydride und Carbonsäuren sowie Acrylate (Michael-Addition) aber auch Wirkstoffe jeder Art können auf diese Weise auf der Oberfläche immobilisiert werden.

In weiteren bevorzugten Umsetzungsprodukten steht ein Teil der Reste R¹ für Ammonioalkyl, und ein Teil der Reste R¹ für einen oder mehrere davon verschiedene Reste. Das Umsetzungsprodukt besitzt neben den kationischen Gruppen, die der Anbindung/Haftung auf einer Materialoberfläche dienen, weitere funktionelle Gruppen. Diese können genutzt werden, um unterschiedliche funktionelle Gruppen auf einer Oberfläche zu verankern. Dieses können Biomoleküle, wie Wirkstoffe, z. B. Bakterizide, Insektizide oder Medikamente sein, die gegebenenfalls erst nach hydrolytischen oder enzymatischen Abbau durch Freisetzung aktiv werden.

Die Erfindung betrifft in einem weiteren Aspekt ein Verfahren zur Modifizierung von Oberflächen, bei dem man die Oberfläche mit einem vorstehend beschriebenen Umsetzungsprodukt, insbesondere einem solchen, in dem zumindest ein Teil der Reste R¹ für Ammonioalkyl steht, in Kontakt bringt. Bei den Oberflächen, die auf diese Weise behandelt werden können, kann es sich um solche aus beliebigen Materialien handeln, z. B. Glas, keramische Werkstoffe, Metalle, Kunststoffe, wie Polyolefine, Polystyrole, schlagzäh modifizierte Polystyrole. Es können auch die Oberflächen von Fasern oder Filamenten behandelt werden. Es ist ein besonderer Vorteil der Erfindung, dass die Beschichtung oder die Applikation des Umsetzungsprodukts aus einer wässrigen Lösung erfolgen kann und keiner komplizierten Vorbereitung und Ausrüstung bedarf wie beispielsweise die Oberflächenbehandlung mit Aminosilanen.

Neben der direkten Oberflächenbeschichtung auch als Schlichten und Avivagen von Fasern ergeben sich für die so dargestellten Polymere Anwendungen als Dispergatoren und (reaktive) Emulgatoren. Weitere Anwendungen im Bereich der polymeren Additive betreffen Haftvermittler, Klebstoffe, Haftklebstoffe und die Immobilisierung von Wirkstoffen.

Die Erfindung wird durch die beigefügte Figur und die nachstehenden Beispiele näher veranschaulicht.

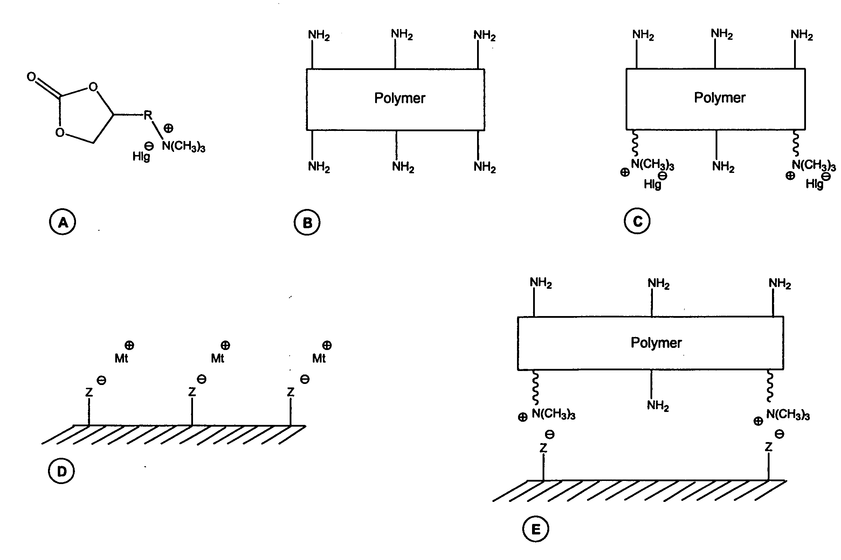

Fig. 1 zeigt schematisch die Herstellung einer erfindungsgemäß modifizierten polykationischen Materialoberfläche. (A) bezeichnet die eingesetzte erfindungsgemäße Verbindung der Formel I (worin R z. B. für -CH₂-OCONH-(CH₂)₃- und Hlg⁻ für ein Halogenid steht) mit einem Ammonium-Substituenten. Die Umsetzung mit dem Polyamin (B) führt zum Umsetzungsprodukt (C). (D) zeigt eine Materialoberfläche mit negativen Oberflächenladungen Z⁻, die locker mit Kationen Mt⁺ assoziiert sind. Bei Behandlung der Materialoberfläche (D) mit dem Umsetzungsprodukt (C) wird das Salz Mt⁺ Hlg⁻ verdrängt und es bildet sich aufgrund der vielfachen Ionenpaarwechselwirkung eine starke Haftung des Polymers zur Oberfläche aus. Am Polymer stehen Aminogruppen (-NH₂) für weitere Modifizierungen zur Verfügung.

### Beipiel 1:

Die Herstellung der erfindungsgemäßen Verbindungen erfolgt nach folgendem Schema:

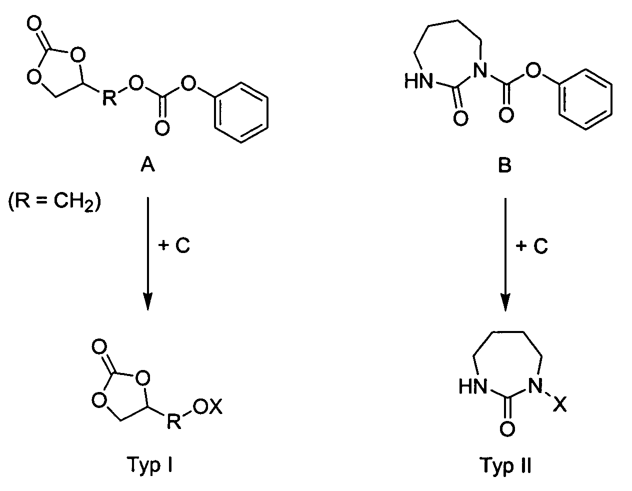

Die Komponente A kann nach der von J. F. G. A. Jansen, A. A. Dias, M. Dorschu, B. Coussens, Macromolecules 2003, 36, 2861-3873 beschriebenen Vorschrift erhalten werden.

Die Komponente B (2-Oxo-[1,3]diazepan-1-kohlensäurephenylester) wurde wie folgt hergestellt: Zu einer Lösung von Tetramethylenharnstoff (1 Äquivalent) in Dichlormethan wurden zunächst 1,5 Äquivalente Triethylamin und danach bei 0 bis 50 °C langsam Phenylchloroformiat (1,5 Äquivalente) zugegeben. Nach beendeter Zugabe rührte man weitere 30 min. bis 2 h. Danach wurde abgekühlt, der Niederschlag (Amin-Hydrochlorid) abfiltriert und mit wenig Lösungsmittel gewaschen. Das Produkt wurde durch Säulenchromatographie gereinigt (Laufmittel: Diethylether / Essigsäureethylester = 1/1) und bei Raumtemperatur im Vakuum (10⁻² mbar) getrocknet. Ausbeute: 90% d. Th.

In einem Kolben geeigneter Größe, der mit Rückflusskühler und regelbarem Rührer versehen ist, gibt man eine der in Tabelle 1 angegebenen Komponenten C in der angegebenen Menge (in Äquivalenten) bei der angegebenen Temperatur unter Rühren zu einer vorgelegten Lösung der Komponente A (zur Herstellung von Verbindungen der Formel I) bzw. Komponente B (zur Herstellung von Verbindungen der Formel II) in einem geeigneten Lösungsmittel (z. B. Chloroform, Dichlormethan, Essigsäureethylester, Dimethylsulfoxid, Dimethylacetamid, Dimethylformamid). Anschließend wird die Lösung über die in Tabelle 1 angegebene Zeit gerührt. Das Produkt wird nach üblichen Verfahren isoliert (Abdestillieren der flüchtigen Bestandteile, Ausfällen und/oder Chromatographie).

**Tabelle 1**

| Nr. | Komponente C | Herstellung von Typ I | | | Herstellung von Typ II | | |
|---|---|---|---|---|---|---|---|
| | | Äq. | Temp. [°C] | Zeit [h] | Äq. | Temp. [°C] | Zeit [h] |
| 1 | Alkylamin | 1,0 - 1,5 | 0 - 25 | 1 - 48 | 1 - 5 | 0 - 50 | 1 - 48 |
| 2 | Fluoralkylamin | 1,0 - 1,5 | 0 - 25 | 1 - 48 | 1 - 5 | 0 - 50 | 1 - 48 |
| 3 | Aminopolysiloxan | 1,0 - 1,5 | 0 - 25 | 1 - 48 | 1 - 5 | 0 - 50 | 1 - 48 |
| 4 | Polyoxyethylen monoamin | 1,0 - 1,5, | 0 - 25 | 1 - 48 | 1. - 5 | 0 - 50 | 1 - 48 |
| 5 | α-Aminoalkyl-ω-alkoxypolyethylenglycol | 1,0 - 1,5 | 0 - 25 | 1 - 48 | 1 - 5 | 0 - 50 | 1 - 48 |
| 6 | Aminoalkohol | 1,0 - 1,5 | 0 - 25 | 1 - 48 | 1 - 5 | 0 - 50 | 1 - 48 |
| 7 | Diamin | - | - | - | 5 - 10 | 0 - 50 | 1 - 48 |
| 8 | (Alkyl)acrylsäure aminoalkylester | 1 ,0 - 1 ,5 | 0 - 25 | 1 - 48 | 1 - 5 | 0 - 50 | 1 - 48 |
| 9 | Aminomonosaccharid (Glucosamin) | 1,0 - 1,5 | 0 - 25 | 1 - 48 | 1 - 5 | 0 - 50 | 1 - 48 |
| 10 | Aminoalkylphosphonsäure | 1,0 - 1,5 | 0 - 25 | 1 - 48 | 1 - 5 | 0 - 50 | 1 - 48 |
| 11 | Phosphorsäure monoaminoalkylester | 1,0 - 1,5 | 0 - 25 | 1 - 48 | 1 - 5 | 0 - 50 | 1 - 48 |
| 12 | Aminoalkansulfonsäure | 1,0 - 1,5 | 0 - 25 | 1 - 48 | 1 - 5 | 0 - 50 | 1 - 48 |
| 13 | Aminoalkyl-trialkylammoniumsalz | 1,0 - 1,5 | 0 - 25 | 1 - 48 | 1 - 5 | 0 - 50 | 1 - 48 |

### Beispiel 2: Anwendungsbeispiele für Verbindungen des Typs I und Typs II

In einem Kolben geeigneter Größe, der mit Rückflusskühler und regelbarem Rührer versehen ist, gibt man eine der in Tabelle 2 angegebenen Komponenten D, die einer der Komponenten C entspricht oder ein stickstoffhaltiges Polymer oder ein Polyol ist, in der angegebenen Menge (in Äquivalenten, bezogen auf die Zahl der funktionellen Gruppen im Polymer) bei der angegebenen Temperatur unter Rühren zu einer Lösung einer Verbindung der Formel I oder II in einem geeigneten Lösungsmittel (z. B. Wasser, Chloroform, Dichlormethan, Essigsäureethylester, Dimethylsulfoxid, Dimethylacetamid, Dimethylformamid). Anschließend wird die Lösung über die in Tabelle 2 angegebene Zeit gerührt. Das Produkt wird nach üblichen Verfahren isoliert, z. B. durch Waschen, Abdestillieren der flüchtigen Bestandteile, Ausfällen, Chromatographie.

**Tabelle 2**

| Nr. | Komponente D | Verbindung I | | | Verbindung II | | |
|---|---|---|---|---|---|---|---|
| | | Äq. | Temp. [°C] | Zeit [h] | Äq. | Temp. [°C] | Zeit [h] |
| 1 | Komponente C | 0,1 - 5 | 20 - 100 | 1 - 48 | 0,1 - 5 | 75 - 150 | 1 - 48 |
| 2 | Polyallylamin | 0,1 - 5 | 20 - 100 | 1 - 48 | 0,1 - 5 | 75 - 150 | 1 - 48 |
| 3 | Polyethylenimin | 0,1 - 5 | 20 - 100 | 1 - 48 | 0,1 - 5 | 75 - 150 | 1 - 48 |
| 4 | Chitosan | 0,1 - 5 | 20 - 100 | 1 - 48 | 0,1 - 5 | 75 - 150 | 1 - 48 |
| 5 | Hercosett-artige Verbindungen | 0,1 - 5 | 20 - 100 | 1 - 48 | 0,1 - 5 | 75 - 150 | 1 - 48 |
| 6 | Polyaminostyrol | 0,1 - 5 | 20 - 100 | 1 - 48 | 0,1 - 5 | 75 - 150 | 1 - 48 |
| 7 | Proteine | 0,1 - 5 | 20 - 100 | 1 - 48 | 0,1 - 5 | 75 - 150 | 1 - 48 |
| 8 | Polyol | - | - | - | 0,1 - 5 | 75 - 150 | 1 - 48 |

## Patentansprüche

1. Verbindung der Formel I oder II 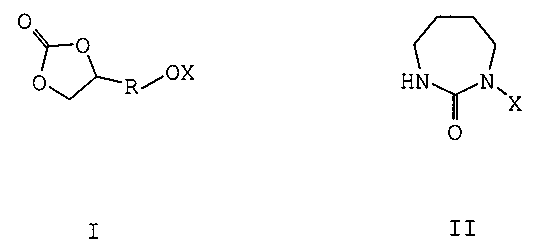 worin
R für C₁-C₁₂-Alkylen steht;
X für CO-NH-R¹ steht; und
R¹ für C₁-C₃₀-Alkyl, C₁-C₃₀-Halogenalkyl, C₁-C₃₀-Hydroxyalkyl, C₁-C₆-Alkyloxy-C₁-C₃₀-alkyl, C₁-C₆-Alkylcarbonyloxy-C₁-C₃₀-alkyl, Amino-C₁-C₃₀-alkyl, Mono- oder Di(C₁-C₆-alkyl)amino-C₁-C₃₀-alkyl, Ammonio-C₁-C₃₀-alkyl, Polyoxyalkylen-C₁-C₃₀alkyl, Polysiloxanyl-C₁-C₃₀-alkyl, (Meth)acryloyloxy-C₁-C₃₀-alkyl, Sulfono-C₁-C₃₀-alkyl, Phosphono-C₁-C₃₀-alkyl, Di(C₁-C₆-alkyl)phosphono-C₁-C₃₀-alkyl, Phosphonato-C₁-C₃₀-alkyl, Di(C₁-C₆-alkyl)phosphonato-C₁-C₃₀-alkyl oder einen Saccharidrest steht.

2. Verbindung nach Anspruch 1, worin R¹ für
-(CH₂)ₙ-CH₃,
-(CH₂)ₙ-(CF₂)ₘ-CF₃,
-(CH₂)ₙ-[Si(CH₃)₂-O]ₚ-H,
-(CH₂)ₙ-(O-CH₂-CH₂)ₚ-O-(CH₂)ₘ-H,
-R²-OH,
-R²-NH₂,
-R²-NR³₃⁺ Y⁻,
-R²-OCO-C(R⁴)=CH₂,
-R²-SO₃H,
-R²-PO₃H₂,
-R²-OPO₃H₂
oder einen Saccharidrest steht,
wobei R² für C₁-C₁₈-Alkylen, R³ für C₁-C₁₈-Alkyl oder Benzyl und R⁴ für Wasserstoff oder Methyl steht,
Y für ein Äquivalent eines Anions steht,
n und m unabhängig voneinander für eine ganze Zahl von 0 bis 12 stehen; und
p für eine ganze Zahl von 1 bis 100 steht.

3. Umsetzungsprodukt einer Verbindung nach Anspruch 1 mit einem Polymer, das über funktionelle Gruppen verfügt, die unter Hydroxygruppen, primären und sekundären Aminogruppen ausgewählt sind.

4. Umsetzungsprodukt nach Anspruch 3, wobei das Polymer ausgewählt ist unter Polyvinylamin, Polyallylamin, Polyethylenimin, Chitosan, Polyamid-Epichlorhydrin-Harzen, Polyaminostyrol, Peptiden oder Proteinen.

5. Umsetzungsprodukt nach Anspruch 3 oder 4, wobei zumindest ein Teil der Reste R¹ für Ammonioalkyl steht.

6. Umsetzungsprodukt nach Anspruch 5, wobei ein Teil der Reste R¹ für von Ammonioalkyl verschiedene Reste steht.

7. Verfahren zur Modifizierung von Biomolekülen, Polymeren oder Oberflächen, die über funktionelle Gruppen verfügen, welche unter Hydroxygruppen, primären und sekundären Aminogruppen ausgewählt sind, bei dem man das Biomolekül, Polymer oder die Oberfläche unter Bedingungen mit einer Verbindung nach Anspruch 1 in Kontakt bringt, dass die funktionellen Gruppen unter Ausbildung einer kovalenten Bindung mit der Verbindung der Formel I oder II reagieren.

8. Verfahren zur Modifizierung von Oberflächen, bei dem man die Oberfläche mit einem Umsetzungsprodukt nach einem der Ansprüche 3 bis 6 in Kontakt bringt.

9. Verwendung des Umsetzungsprodukts nach einem der Ansprüche 3 bis 6 als Avivagemittel, Dispergator, Emulgator, Haftvermittler, Klebstoff, Haftklebstoff oder zur Immobilisierung von Wirkstoffen.
